(19) **Europäisches Patentamt / European Patent Office / Office européen des brevets**

(11) **EP 3 761 063 B1**

(12) **FASCICULE DE BREVET EUROPEEN**

(45) Date de publication et mention de la délivrance du brevet:
**11.02.2026 Bulletin 2026/07**

(21) Numéro de dépôt: **19183638.6**

(22) Date de dépôt: **01.07.2019**

(51) Classification Internationale des Brevets (IPC):
**G01S 19/34** (2010.01)     **G01S 19/52** (2010.01)
**G01S 19/19** (2010.01)

(52) Classification Coopérative des Brevets (CPC):
**G01S 19/19; G01S 5/017; G01S 19/34; G01S 19/52**

(54) **INSTRUMENT PORTABLE DE GESTION D'UNE ACTIVITE SPORTIVE OU DE BIEN-ETRE**

TRAGBARES INSTRUMENT ZUR STEUERUNG EINER SPORT- ODER FITNESS-AKTIVITÄT

PORTABLE INSTRUMENT FOR MANAGING A SPORTS OR FITNESS ACTIVITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**06.01.2021 Bulletin 2021/01**

(73) Titulaire: **The Swatch Group Research and Development Ltd**
**2074 Marin (CH)**

(72) Inventeurs:
- **NICOLAS, Cédric**
  **2000 Neuchâtel (CH)**
- **AMINIAN, Kamiar**
  **1814 La Tour-de-Peilz (CH)**
- **CABEZA DE PABLO, Joaquin**
  **1020 Renens (CH)**
- **SAVARY, Martin**
  **1400 Yverdon-les-Bains (CH)**
- **SOLTANI, Abolfazl**
  **1007 Lausanne (CH)**
- **DEJNABADI, Hooman**
  **1350 Orbe (CH)**

(74) Mandataire: **ICB SA**
**Faubourg de l'Hôpital, 3**
**2001 Neuchâtel (CH)**

(56) Documents cités:
**US-A1- 2013 271 314    US-A1- 2018 356 534**
**US-B1- 6 532 432**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

DOMAINE TECHNIQUE DE L'INVENTION

L'invention concerne un instrument portable, tel qu'une montre, muni d'un dispositif de contrôle ou gestion d'une activité sportive ou de bien-être d'une personne portant l'instrument portable en activité.

L'invention concerne aussi un procédé de gestion d'une activité sportive ou de bien-être au moyen de l'instrument portable en fonction.

ETAT DE LA TECHNIQUE

Les vitesses de marche ou allures sont parmi les paramètres les plus importants pour caractériser la mobilité journalière des personnes. Par exemple dans des applications sportives, la vitesse peut être utilisée pour évaluer les athlètes et ainsi préparer des séances personnalisées d'entraînement, dans le but d'améliorer la performance de chaque athlète et de réduire le risque de blessures. Dans des applications médicales, la vitesse est utilisée pour évaluer la santé d'une personne, dans le but d'aider les médecins afin d'établir un diagnostic, prédire et prévenir de nombreuses maladies, telles que les pathologies cardiovasculaires ou le diabète ou le surpoids.

Un système de navigation global par satellites (GNSS) est un système de base largement utilisé pour mesurer par exemple la vitesse de marche d'une personne. Un tel système GNSS est précis et de nombreux instruments portables ont été conçus pour intégrer un tel transpondeur, dont les mesures peuvent être utilisées pour calculer la vitesse de marche d'une personne même dans des conditions réelles. Cependant, il y a certains endroits où le signal GNSS est faible ou pourrait même être perdu en raison du manque de couverture par satellites, comme à l'intérieur de tunnels, près de hauts bâtiments, dans des vallées étroites. De plus, un transpondeur GNSS consomme beaucoup d'énergie électrique. De ce fait, il est préférable de l'utiliser de façon sporadique plutôt que de façon continue pour réduire la consommation électrique de l'instrument portable qui le comprend.

La demande de brevet WO 2018/106319 A1 décrit un instrument portable, tel qu'un téléphone portable ou une montre intelligente, pour estimer en temps réel des paramètres de mouvement d'une personne, tels qu'une vitesse ou une cadence de marche ou de course. L'instrument comprend un transpondeur GNSS avec un filtre de Kalman pour déterminer une première vitesse dérivée des positions GNSS de la personne, une deuxième vitesse dérivée des décalages Doppler des signaux GNSS et un nombre de pas observés de l'utilisateur. L'instrument comprend encore des unités de détection de mouvement, qui peuvent fournir une vitesse dérivée des positions GNSS et une vitesse dérivée des décalages Doppler GNSS. Cependant l'utilisation du transpondeur GNSS de l'instrument est utilisé pendant de grandes périodes de temps pour déterminer une vitesse ou cadence de marche de la personne, ce qui conduit à une forte consommation électrique et constitue un inconvénient.

La demande de brevet WO 2012/045484 A1 décrit un système podomètre calibré par GPS. Le système peut être porté par une personne, tel qu'une montre de sport. Le système comprend un récepteur GNSS conçu pour obtenir la position et/ou la vitesse de la personne et un podomètre pour le comptage des pas effectués par la personne. Les données du récepteur GNSS sont utilisées pour calibrer le podomètre chaque fois que l'utilisateur est déterminé à parcourir une distance supérieure à une valeur de distance prédéfinie pendant une période au cours de laquelle les signaux obtenus par le récepteur GNSS sont précis. Comme pour le précédent document, le récepteur GNSS de l'instrument est utilisé pendant de grandes périodes de temps pour déterminer une vitesse ou cadence de marche de la personne, ce qui conduit à une forte consommation électrique et constitue un inconvénient.

Le brevet US 7,245,254 B1 décrit un dispositif d'exercice électronique pour contrôler l'activité ou mobilité d'une personne. Le dispositif électronique calcule en continu les pas de l'utilisateur à l'aide d'un circuit GPS de détermination de la position et d'un instrument informatique en exécutant un processus de calibration itérative. Le dispositif comprend donc un circuit GPS, un podomètre, un accéléromètre, un capteur de contrôle du pouls et un capteur de température. Quand les signaux de satellite GPS sont accessibles, le circuit GPS corrige l'erreur accumulée du podomètre et/ou de l'accéléromètre. Comme pour les précédents documents, le circuit GPS de l'instrument est utilisé pendant de grandes périodes de temps pour déterminer en continu des pas ou des paramètres physiques de la personne, ce qui conduit à une forte consommation électrique et constitue un inconvénient.

La demande de brevet US 2018/0356534 A1 décrit un dispositif portable, tel qu'une montre-bracelet, qui comprend une unité de détection GPS de position, une unité à capteurs, et une unité de traitement liée à l'unité de détection de position et à l'unité à capteurs. Le dispositif est utilisé dans un système support d'exercices. Le dispositif est porté au poignet d'un utilisateur lors d'une course et est susceptible de fournir un affichage de la position, de la vitesse, de la distance parcourue notamment. Il comprend au moins un capteur de mouvement relié à l'unité de traitement et un module récepteur GNSS. Le module GPS n'est activé que par périodes courtes par l'unité de traitement et un contrôle de l'état du mouvement de l'utilisateur est effectué pour arrêter le module GPS si le mouvement ne change pas d'état. Il n'est pas prévu d'adapter les temps d'activation ou de désactivation du module GPS.

# EP 3 761 063 B1

La demande de brevet US 2013/0271314 A1 décrit un dispositif portable, qui peut être utilisé pour une activité sportive avec un module récepteur GNSS. Le dispositif portable comprend encore un capteur de mouvement relié à une unité de calcul, qui est relié aussi au module récepteur GNSS. Un module contrôleur est prévu pour instruire l'unité de calcul pour effectuer le calcul de vitesse des mesures du capteur de mouvement quand le module récepteur GNSS est dans un état faible puissance pendant des intervalles de temps. Il n'est pas prévu d'adapter les temps d'activation ou de désactivation du module GPS.

## RESUMÉ DE L'INVENTION

L'invention a donc pour but de pallier les inconvénients cités ci-dessus avec un instrument portable muni d'un dispositif de contrôle ou gestion d'une activité sportive ou de bien-être d'une personne portant l'instrument portable et en réduisant le temps de fonctionnement d'un module récepteur GNSS du dispositif afin de réduire la consommation électrique tout en déterminant précisément la mobilité quotidienne de la personne par le dispositif.

A cet effet, l'invention concerne un instrument portable muni d'un dispositif de contrôle ou gestion d'une activité sportive ou de bien-être d'une personne portant l'instrument portable, qui comprend les caractéristiques de la revendication indépendante 1.

Des formes d'exécution particulières de l'instrument portable sont définies dans les revendications dépendantes 2 à 6.

Un avantage de l'instrument portable muni du dispositif de contrôle réside dans le fait d'une activation par courtes périodes temporelles du module récepteur GNSS pour déterminer des changements de cadence ou profil de marche d'une personne. Ceci permet de réduire la consommation électrique du dispositif de contrôle tout en calibrant le dispositif de contrôle de l'instrument portable pour la personne l'utilisant à long terme. Ainsi le dispositif de contrôle apprend le profil de mobilité quotidienne de la personne pour avoir une calibration personnalisée et de manière auto-adaptive par l'opération de calibration avec l'activation par courtes périodes temporelles du module récepteur GNSS pour déterminer précisément les données de vitesse en particulier.

Avantageusement, avant une nouvelle activation du module récepteur GNSS, un temps de désactivation doit être dépassé indépendamment de la réception de nouveaux modèles ou profils de marche ou de démarche. Pour ce faire, le temps d'activation du module récepteur GNSS est inférieur d'à peu près 5 fois au temps de désactivation du module récepteur GNSS.

A cet effet, l'invention concerne également un procédé de gestion d'une activité sportive ou de bien-être d'une personne au moyen de l'instrument portable, qui comprend les caractéristiques mentionnées dans la revendication indépendante 7.

Des étapes particulières du procédé de gestion d'une activité sportive ou de bien-être d'une personne sont définies dans les revendications dépendantes 8 à 11.

## BRÈVE DESCRIPTION DES FIGURES

Les buts, avantages et caractéristiques d'un instrument portable ou d'un procédé de gestion d'une activité sportive ou de bien-être d'une personne apparaîtront mieux dans la description suivante sur la base d'au moins une forme d'exécution non limitative illustrée par les dessins sur lesquels :

- la figure 1 est une vue en perspective d'une personne portant l'instrument portable sous la forme d'une montre-bracelet selon l'invention,

- la figure 2 représente une vue simplifiée d'une personne portant l'instrument portable, qui comprend selon une variante de réalisation trois parties selon l'invention,

- la figure 3 est une vue schématique en perspective d'un chemin suivi par une personne munie de l'instrument portable durant une marche ou une course à pieds,

- la figure 4 représente un schéma bloc simplifié des composants électroniques du dispositif de contrôle ou de gestion d'une activité sportive ou de bien-être d'une personne, de l'instrument portable selon l'invention,

- la figure 5 représente différentes étapes d'un procédé de gestion d'une activité sportive ou de bien-être d'une personne portant l'instrument portable selon l'invention,

- la figure 6 représente un graphique d'une diminution des probabilités exponentielles pour différentes valeurs de $\beta$ pour la mise en marche du module récepteur GNSS du dispositif de contrôle selon l'invention,

- la figure 7 représente un diagramme comparatif des résultats de la stratégie GNSS pour une personne dans le temps avec d'une part le module récepteur GPS activé en permanence et d'autre part le module récepteur GPS activé partiellement suite à des modifications de profil de marche de la personne, et

- la figure 8 représente un diagramme comparatif de l'erreur relative de vitesse en fonction du pourcentage de personnes utilisant l'instrument portable personnalisé avec d'une part le module récepteur GPS activé en permanence et d'autre part le module récepteur GPS activé partiellement suite à des modifications de profil de marche des personnes.

DESCRIPTION DETAILLEE DE L'INVENTION

Dans la description suivante, tous les composants électroniques d'un instrument portable, qui est muni d'un dispositif de contrôle d'une activité sportive ou de bien-être d'une personne portant l'instrument portable, qui sont bien connus d'un homme du métier dans ce domaine technique, ne sont décrits que de manière simplifiée. Il est à noter qu'il est cherché de gérer une activité sportive ou de bien-être d'une personne, c'est-à-dire un déplacement à pieds. Il doit être compris qu'en ne définissant qu'une marche ou démarche de la personne, cela comprend aussi une course à pieds par exemple.

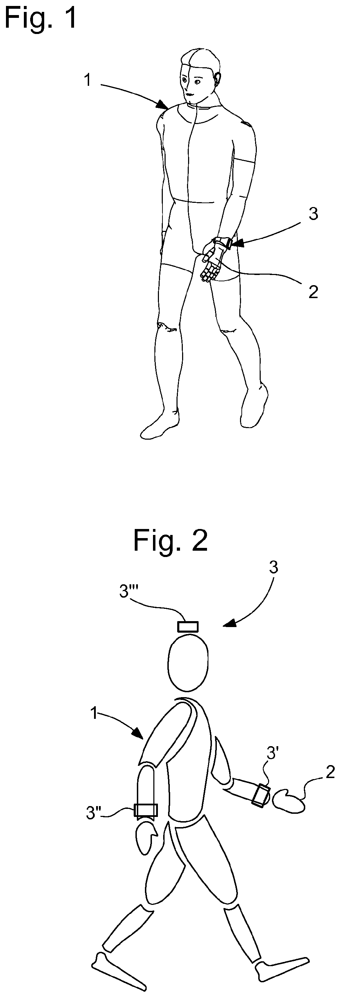

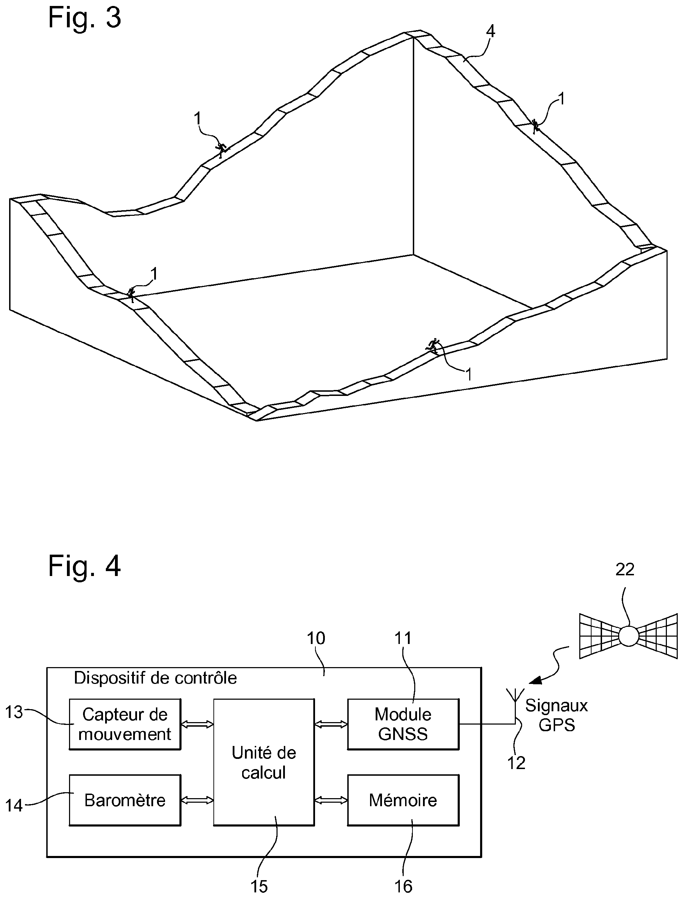

La figure 1 représente une personne 1 en position debout les pieds en contact du sol. La personne 1 porte un instrument portable 3, qui est dans cette forme d'exécution une montre-bracelet qu'il porte à son poignet gauche 2 par exemple. La montre-bracelet 3 est pourvue d'un dispositif de contrôle ou gestion d'une activité sportive ou de bien-être expliqué ci-après. La montre intelligente 3 est configurée par son dispositif de contrôle pour surveiller l'activité et déduire divers paramètres de la personne 1 pendant qu'elle se déplace à pieds (c.-à-d. en courant ou en marchant) sur une trajectoire 4, dont les variations d'altitude ou de pente sont volontairement exagérées comme représenté en figure 3. Le dispositif de contrôle comprend au moins un capteur de mouvement, qui peut être un accéléromètre à un ou deux ou de préférence trois axes de mesure, et encore par exemple au moins un capteur de pression, tel qu'un baromètre ou altimètre pour déterminer l'altitude ou la pente lors d'une marche ou course sur une trajectoire 4 ou chemin dans la nature ou en zone urbaine.

A la figure 2 selon une variante d'exécution, la personne 1 porte un instrument portable 3, qui peut être composé de trois parties 3', 3", 3"' non directement reliées mécaniquement, mais connectées électriquement ou sans fil. Ces trois parties comprennent tous les composants du dispositif de contrôle. Dans une première partie 3' de l'instrument portable 3, qui est fixée par exemple au poignet gauche 2 de la personne 1 sous la forme d'un bracelet ou d'une montre-bracelet, il est prévu au moins un premier capteur du dispositif de contrôle. Cette première partie 3' peut comprendre une unité de calcul pour le traitement de toutes les données ou mesures des trois parties de l'instrument portable 3. Ce premier capteur peut être un capteur de mouvement avec au moins un accéléromètre par exemple à trois axes de mesure. Le capteur de mouvement peut aussi être un capteur inertiel de mouvement à 9 axes ayant un accéléromètre triaxial, un gyroscope triaxial et un capteur magnétique triaxial. Dans une seconde partie 3" de l'instrument portable 3, qui est fixée par exemple au poignet droit de la personne 1 sous la forme d'un bracelet, il est prévu au moins un second capteur du dispositif de contrôle. Ce second capteur peut être un capteur de pression, tel qu'un altimètre ou baromètre. Finalement dans une troisième partie 3"' de l'instrument portable 3, qui est montée sur la tête de la personne 1 dans un bandeau ou un casque, il est prévu le module récepteur GNSS, qui est utilisé comme une méthode de référence.

Il est à noter que le capteur de mouvement peut aussi être un capteur inertiel à 10 axes avec un accéléromètre triaxial, un gyroscope triaxial, un capteur magnétique triaxial, et un baromètre pour déterminer des coordonnées locales et la pente du chemin emprunté par la personne 1. De plus, le placement d'un capteur inertiel à 10 axes à chaque pied permettrait d'avoir une mesure plus simple et plus précise du nombre de pas et de la cadence de marche ou course de la personne portant l'instrument portable. Le placement du module récepteur GNSS de référence sur la tête est la meilleure position pour ne pas être dépendant du mouvement des bras ou jambes.

La figure 4 représente de manière plus précise les différents composants électroniques du dispositif de contrôle 10 ou de gestion d'une activité sportive ou de bien-être d'une personne de l'instrument portable selon l'invention. Le dispositif de contrôle 10 comprend au moins un capteur de mouvement 13 relié à une unité de calcul 15, qui peut être un microcontrôleur cadencé par un oscillateur intégré non représenté. Le dispositif de contrôle 10 comprend encore un module récepteur GNSS 11 contrôlé par l'unité de calcul 15 pour l'activer ou le désactiver. Le dispositif de contrôle 10 peut encore comprendre un capteur de pression 14, qui est un baromètre ou un altimètre, relié à l'unité de calcul 15, et au moins une mémoire 16 reliée à l'unité de calcul 15, telle qu'une mémoire non volatile 16 capable de mémoriser différentes mesures effectuées par le ou les capteurs 13, 14 ou des mesures reçues par des signaux GPS de satellites visibles 22 par une antenne 12 liée au module récepteur GNSS 11. Le dispositif de contrôle 10 est généralement alimenté par une batterie non représentée de l'instrument portable pour sa mise en fonction.

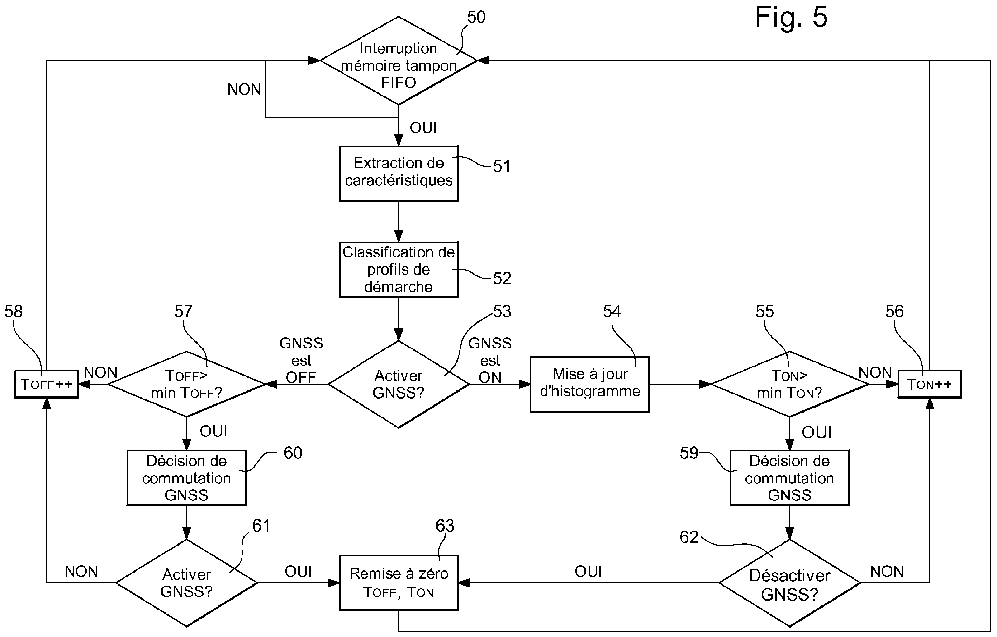

L'unité de calcul 15, qui est un microcontrôleur, peut comprendre en plus de l'oscillateur un premier compteur pour déterminer un temps d'activation du module récepteur GNSS 11 et un second compteur pour déterminer un temps de

4

désactivation du module récepteur GNSS 11. Un premier seuil de commutation est prévu en liaison au premier compteur et un second seuil de commutation est prévu en liaison au second compteur comme expliqué ci-après en référence au procédé de gestion en figure 5. Le temps d'activation et le temps de désactivation déterminés dans l'unité de calcul 15 ou microcontrôleur peuvent être définis par un autre moyen qu'un compteur de temps.

L'unité de calcul 15, tel que le microcontrôleur, peut avoir mémorisé un algorithme de calcul pour l'estimation de vitesse ou de cadence du déplacement de la personne. Il peut aussi être prévu de mémoriser l'algorithme dans la mémoire non volatile 16. Cet algorithme incorporé ici par référence a été présenté par Mr. Abolfazl Soltani, et al. dans l'article intitulé "Real-world gait speed estimation using wrist sensor: A personalized approach.", et présenté dans IEEE journal of biomedical and health informatics (2019). Des données de vitesse sont donc collectées et mémorisées de préférence dans la mémoire non volatile 16 ou une mémoire volatile pour caractériser des styles ou profils de marche ou course d'une personne dans la vie quotidienne en utilisant des signaux de satellites 22 et des signaux des capteurs 13, 14. Il est ainsi possible de définir un modèle personnalisé dans le but de n'activer le module récepteur GNSS 11 que lors de variations de mouvement ou de pression s'écartant des variations déjà connues précédemment et mémorisées. Ceci permet de réduire la consommation générale du dispositif de contrôle 10 comme il est alimenté par une petite batterie.

Pour mieux comprendre le fonctionnement du dispositif de contrôle, il est fait référence maintenant au procédé de gestion d'une activité sportive ou de bien-être d'une personne portant l'instrument portable en référence à la figure 5. Comme précédemment indiqué selon la présente invention, la stratégie proposée GNSS est d'activer le module récepteur GNSS à chaque fois qu'il y a de nouveaux modèles ou profils de marche ou démarche d'une personne durant la vie quotidienne. Dans la figure 5 décrite ci-après, le diagramme des différentes étapes de la stratégie intelligente proposée GNSS est représenté. Dans ce cas de figure, le dispositif de contrôle comprend aussi bien le capteur de mouvement que le capteur de pression, mais en règle générale au moins le capteur de mouvement est nécessaire.

Interruption de mémoire tampon FIFO 50: dans cette étape, la stratégie intelligente attend pour une interruption de mémoire tampon FIFO indiquant la présence de nouveaux échantillons suite à des variations de mouvement d'un profil de marche non encore mémorisé. Cette mémoire tampon FIFO peut faire partie de la mémoire d'enregistrement de données de vitesse et de différents profils de marche ou démarche.

Extraction de caractéristiques 51 : L'algorithme proposé utilise dans cet exemple un accéléromètre tridimensionnel 3D et un capteur de pression barométrique pour fournir un signal d'accéléromètre 3D (A(t)) et un signal de pression (P(t)). Les signaux sont segmentés toutes les secondes à l'aide d'une fenêtre mobile de 6 secondes avec un chevauchement de 5 secondes pour fournir une accélération segmentée (A[n]) et un signal de pression (P[n]), où n indique le numéro de la fenêtre. Sx [n], Sy [n] et Sz [n] ont été désignés comme des accélérations segmentées le long des trois axes de mesure de l'accéléromètre.

Concernant les fenêtres mobiles toutes les secondes, il s'agit de fenêtres mobiles successives dans le temps de durée chacune de 6 secondes et se chevauchant de 5 secondes chacune avec une fenêtre successive. Ainsi les différentes fenêtres successives sont décalées de 1 seconde à chaque fois. Le cadencement de ces fenêtres mobiles de mesure est obtenu par l'intermédiaire de l'oscillateur du microcontrôleur et une série de diviseurs si nécessaires. Avec ces fenêtres de mesure, il est possible de détecter l'immobilité de la personne, une incertitude sur les variations de mouvement ou la mobilité de la personne. La mobilité ou déplacement de la personne est un paramètre nécessaire mais non suffisant à la commande directe de l'activation du module récepteur GNSS.

Lorsque de nouvelles données du capteur de mouvement, tel que l'accéléromètre, et du capteur de pression, tel que le baromètre, deviennent disponibles, deux caractéristiques sont extraites selon les équations (1) et (2) ci-dessous. Ces caractéristiques sont spécialement choisies car elles permettent de regrouper les différents modèles ou profils de marche et leurs caractéristiques inhérentes (p. ex., course rapide/lente, montée/descente, etc.). Une fenêtre de 6 secondes avec un chevauchement de 5 secondes par rapport à d'autres fenêtres successives est utilisée pour l'extraction des caractéristiques.

$$F_1[n] = -\frac{\sum_{i=1}^q (i-\bar{\imath})\cdot\left(P^i[n]-\bar{P}[n]\right)}{\sum_{i=1}^q (i-\bar{\imath})^2}\cdot F_s \qquad (1)$$

$$F_2[n] = \frac{std(S_y[n])}{\frac{1}{q}\sum_{i=1}^q \left|S_y^i[n]-S_y^{i-1}[n]\right|} \qquad (2)$$

où q est le nombre d'échantillons dans la fenêtre numéro n, $F_s$ est la fréquence d'échantillonnage (500 Hz dans ce cas), et $p^i[n]$ est le i-ième échantillon du vecteur de pression dans la fenêtre numéro n. De plus, P[n] et i sont calculés sur la base des équations (3) et (4). Std signifie une déviation standard où $S_y[n]$ est une valeur d'accélération enregistrée sur l'axe y du capteur. De plus, $S^i_y[n]$ est le i-ième échantillon du vecteur $S_y[n]$.

$$\bar{P}[n] = \frac{1}{q}\sum_{i=1}^{q} P^i[n] \tag{3}$$

$$\bar{i} = \frac{1}{q}\sum_{i=1}^{q} i \tag{4}$$

Classification des modèles ou profils de marche 52 : Le modèle ou profil de marche ou course ou cadence est défini sur la base d'une valeur de F1 et F2 dans une table d'histogramme non représentée. A ce stade, le but est de décider si les données des capteurs contiennent de nouvelles informations pour l'entraînement sur le modèle de vitesse ou pas. A cette fin, une table d'histogramme est conçue où chaque colonne est en relation à F1 et chaque rangée est en relation à F2. La marge sélectionnée (RF1) et la résolution (dF1) pour F1 sont définies par exemple avec RF1 = [-0.07 à +0.07] et dF1 = 0.035. De manière similaire, la marge (RF2) et la résolution (dF2) pour F2 sont définies par exemple avec RF2 = [0 à 5] et dF2 = 0.5. Dans ce cas, l'espace créé par <F1, F2> contient 55 cellules utilisées pour regrouper chaque modèle ou profil de marche ainsi que chaque cellule dans la table d'histogramme montre le nombre d'occurrences des données d'adaptation dans la cellule.

Finalement en utilisant l'équation (5), le nombre d'occurrences est traduit en une valeur de probabilité indiquant la probabilité de mise en marche du module récepteur GNSS si un nouvel échantillon est dans la marge d'une de ces cellules reçues des capteurs.

$$P_i = 2^{-\left(\frac{N_i}{\beta}\right)} \tag{5}$$

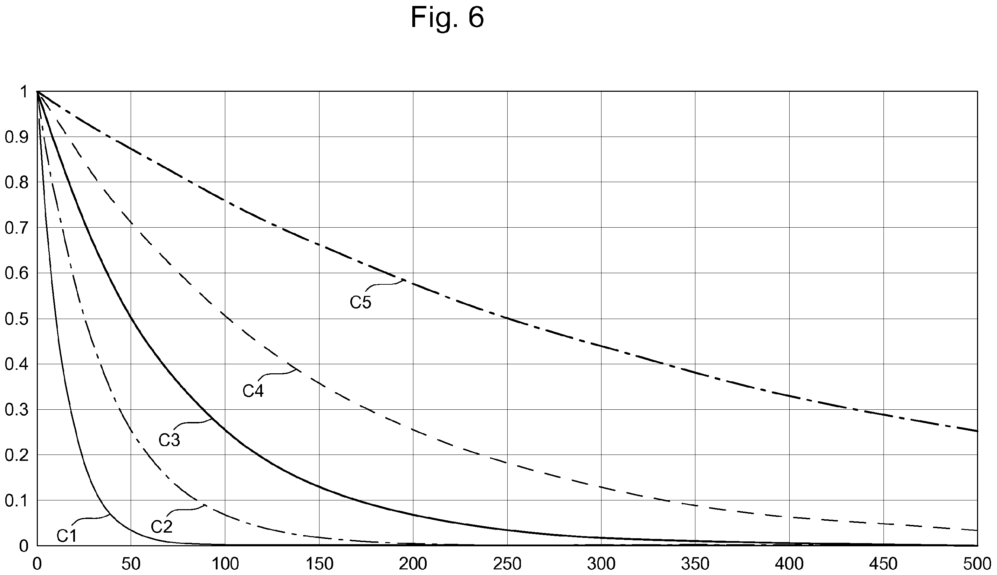

où $N_i$ est le nombre d'occurrences dans chaque cellule et $\beta$ est le nombre de fois, qu'une situation doit apparaître pour atteindre la moitié de la valeur de la courbe exponentielle comme représenté à la figure 6. La courbe c1 est pour $\beta$ égal à 10. La courbe c2 est pour $\beta$ égal à 25. La courbe c3 est pour $\beta$ égal à 50. La courbe c4 est pour $\beta$ égal à 100. La courbe c5 est pour $\beta$ égal à 250. Au début de la personnalisation, la table d'histogramme est remplie de zéros ainsi que toutes les probabilités sont égales à 1.

Contrôle de l'état du GNSS 53 : à cette étape, l'état du module récepteur GNSS (ON/OFF) est analysé pour trouver la bonne ligne d'exécution dans l'algorithme.

Mise à jour d'histogramme 54 : Si le module récepteur GNSS est déjà ON, la table d'histogramme contenant le nombre d'occurrences de chaque modèle ou profil de marche est mise à jour.

$T_{ON}$ > min $T_{ON}$ ? 55 : Chaque fois que l'algorithme détecte que le module récepteur GNSS est ON, le compteur correspondant ($T_{ON}$), qui fait partie de l'unité de calcul ou microcontrôleur, est comparé à un seuil (min $T_{ON}$). $T_{ON}$ contient la quantité de fois consécutives (exprimée en secondes) que le module récepteur GNSS est utilisé. Le seuil min $T_{ON}$ évite que le module récepteur GNSS change son état trop fréquemment, car cela causerait un comportement instable et une consommation de courant plus haute. Il est important de considérer que le temps écoulé entre l'instant où la tension alimente le dispositif de contrôle avec le module récepteur GNSS et les mesures du module récepteur GNSS utiles sont reçues, peut augmenter de plusieurs secondes. Dans le domaine du GNSS, ce temps est connu comme TTFF ("Time To First Fix" en terminologie anglaise) et sa valeur peut grandement changer selon l'état initial du récepteur et les conditions environnementales. Par conséquent, la décision ON pour changer l'état du module récepteur GNSS aura certaines restrictions sur la quantité minimum de fois où le module récepteur GNSS doit rester dans le même état. Ces restrictions sont gouvernées par la valeur du seuil min $T_{ON}$, qui peut être par exemple fixé à 2 minutes, qui est le temps d'activation du module récepteur GNSS.

$T_{ON}$ ++ 56 : si la condition de seuil n'est pas rencontrée ou le module récepteur GNSS reste dans le même état après l'exécution de la décision de commutation du module récepteur GNSS, le compteur $T_{ON}$ est incrémenté.

$T_{OFF}$ > min $T_{OFF}$ ? 57 : chaque fois que l'algorithme détecte que le module récepteur GNSS est OFF, le compteur correspondant ($T_{OFF}$), qui fait partie de l'unité de calcul ou microcontrôleur, est comparé à un seuil (min $T_{OFF}$). $T_{OFF}$ contient la quantité de fois consécutives (exprimée en secondes) où le module récepteur GNSS n'est pas utilisé. Les mêmes restrictions pour éviter le changement d'état du module récepteur GNSS trop rapide est gouverné par la valeur du seuil min $T_{OFF}$, qui peut être par exemple fixé à 10 minutes. Cette valeur seuil (temps de désactivation) peut être défini aussi plus grand pour tenir compte de profils de marche déjà connus et mémorisés, et étant donné qu'il est prévu au moins un temps d'activation du module récepteur GNSS plus petit, par exemple au moins 5 fois plus petit, pour pouvoir au moins déterminer précisément par le module récepteur GNSS activé une distance, une position ou de préférence une vitesse dans une opération de calibration personnelle du dispositif de contrôle.

$T_{OFF}$ ++ 58 : si la condition de seuil n'est pas dépassée ou le module récepteur GNSS reste dans le même état après l'exécution de la décision de commutation du module récepteur GNSS, le compteur $T_{OFF}$ est incrémenté.

Décision de commutation du module récepteur GNSS 59, 60 : sur la base de la probabilité en utilisant l'équation (5), une décision ON est prise si le changement de l'état du module récepteur GNSS est exécuté ou pas. Par exemple, si le module récepteur GNSS est OFF et la probabilité de commutation ON est à 75%, une valeur de probabilité aléatoire dans la marge [0 à 100] est générée par exemple en utilisant une distribution normale. Plus tard, si la probabilité générée aléatoire est plus petite que la probabilité de commutation ON c'est-à-dire 75%, une décision de commuter ON le module récepteur GNSS est générée. Cela prend du sens comme les probabilités plus élevées sont attendues lorsque de nouvelles situations apparaissent et ainsi il est peu probable que la probabilité aléatoire générée a une plus grande valeur. De manière analogue, si le module récepteur GNSS est ON et la probabilité de commutation OFF est à 75%, le module récepteur GNSS serait désactivé (OFF) seulement si la probabilité aléatoire générée est plus grande que 75% dans ce cas. A nouveau, cela prend du sens comme de faibles probabilités sont attendues si le module récepteur GNSS est ON comme des situations sont déjà entraînées ainsi qu'il est peu probable que la probabilité aléatoire générée a une plus petite valeur.

Activer GNSS (ON) ? 61 : si le module récepteur GNSS est OFF, la décision de commutation du module récepteur GNSS est contrôlée pour le commuter ON.

Désactiver GNSS (OFF) ? 62 : si le module récepteur GNSS est ON, la décision de commutation du module récepteur GNSS est contrôlée pour le commuter OFF.

Mettre à zéro $T_{ON}$, $T_{OFF}$ 63 : si la décision de commutation du module récepteur GNSS est affirmative, les compteurs $T_{ON}$, $T_{OFF}$ sont remis à zéro et l'algorithme commencera à attendre à nouveau pour une interruption de la mémoire tampon FIFO à l'étape 50.

En guise de résultats pour analyser la performance de la stratégie intelligente du module récepteur GNSS, il est focalisé sur les paramètres suivants :

Niveau de convergence : L'algorithme RLS ("Recursive Least Square" en terminologie anglaise) est utilisé pour construire un modèle d'estimation de vitesse personnalisé. Le niveau de convergence ou « procédé d'apprentissage » peut être étudié en regardant au moins le premier élément dans la diagonale de la matrice de covariance d'échantillons. On regarde à cette valeur pour étudier la convergence du modèle d'estimation de vitesse comparé au cas où les échantillons du module récepteur GNSS sont toujours utilisés.

Usage du module récepteur GNSS : chaque fois que le module récepteur GNSS est utilisé, un compteur est incrémenté dans le microcontrôleur. Ce compteur est utilisé pour contrôler la quantité d'utilisations du module récepteur GNSS requis par la stratégie intelligente GNSS et étudier la faisabilité et l'impact de réduction de ce temps.

Erreur relative de vitesse : l'erreur relative pour chaque échantillon de vitesse estimée est calculé en utilisant l'expression suivante :

$$Rel.\,Speed\,Error = \left| \frac{\hat{v} - \hat{v}_{ref}}{\hat{v}_{ref}} \right| \cdot 100 \tag{6}$$

où v est la vitesse estimée et $v_{ref}$ est la vitesse de référence GNSS.

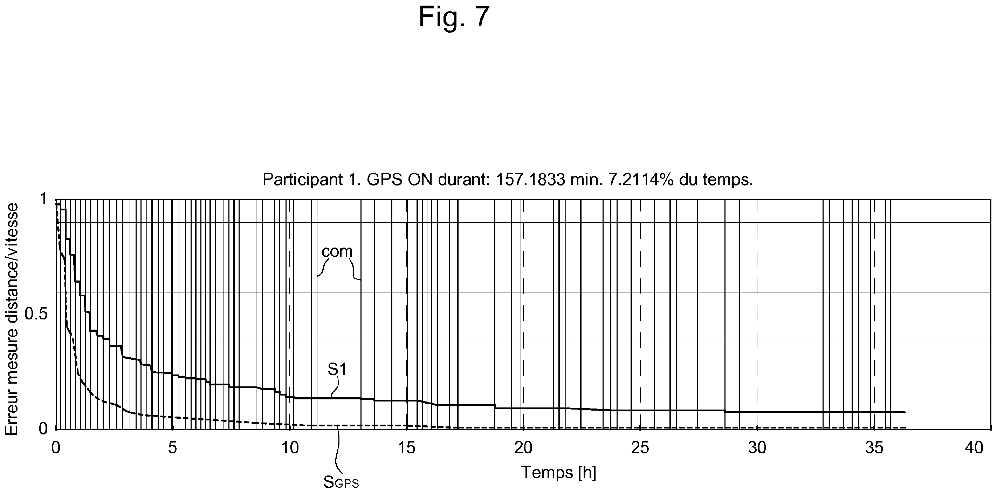

La figure 7 illustre les résultats de l'application de la stratégie intelligente GNSS à un des participants dans une base de données définie M3. Cette base de données contient des données provenant uniquement de situations en cours d'exécution. Les participants n'ont pas de contrainte et portent les capteurs pendant leur entraînement de course normale et leur trajectoire habituelle. Cette base de données est enregistrée dans le dispositif de contrôle porté au poignet du participant 1 par exemple.

Comme il peut être vu dans la figure 7, le module récepteur GNSS du participant 1 est seulement utilisé et activé durant 7.2% de la durée totale, qui est représentée sur environ 36h, c'est-à-dire pendant 157.1833 minutes. Les différents traits verticaux com définissent les instants d'activation du module récepteur GNSS de la présente invention. Ainsi, l'erreur de mesure distance/vitesse du signal S1 du module récepteur GNSS activé par périodes temporelles com est jugé faible et le signal S1 est proche d'un signal $S_{GPS}$ d'un module récepteur GNSS, qui serait activé en continu pendant toute la durée des 36h. Le graphique représente plus précisément le niveau de convergence de la personnalisation (S1), qui est exprimé par le premier élément dans la diagonale de la matrice de covariance des échantillons, et qui est raisonnablement proche au cas où le module récepteur GNSS est toujours activé ($S_{GPS}$).

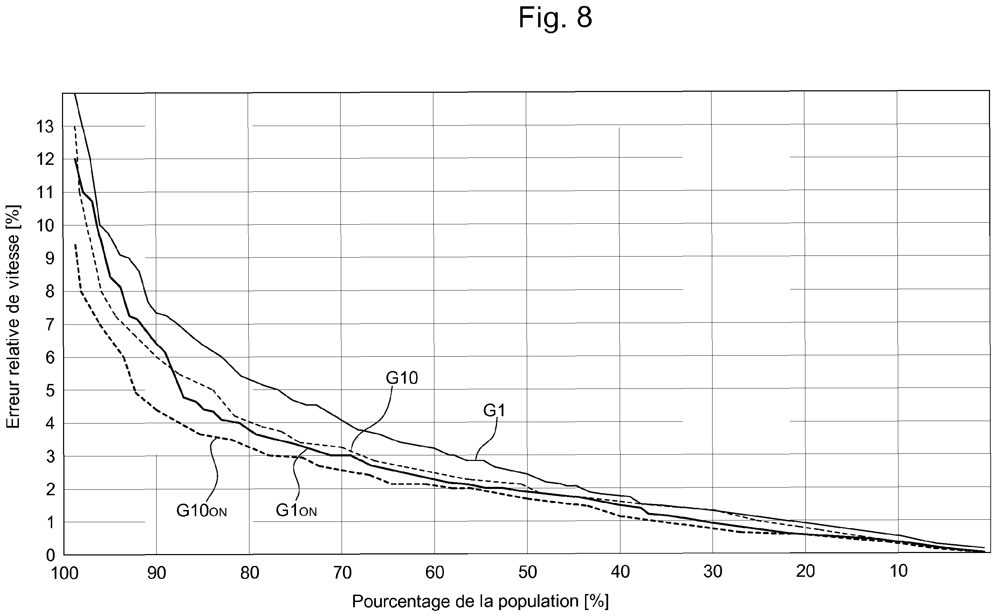

Finalement dans la figure 8, l'erreur relative dans l'estimation de vitesse pour différentes personnes (pourcentage de personnes) est représentée. Pour comparer la performance de la stratégie intelligente GNSS avec le cas où le module récepteur GNSS est utilisé tout le temps, on calcule l'erreur relative en utilisant l'équation (6) pour les deux situations. En effet, lorsque le module récepteur GNSS est toujours utilisé, l'erreur relative comprend seulement l'erreur de poursuite de l'algorithme RLS quand on essaie de suivre la référence de vitesse. D'autre part, quand la stratégie intelligente GNSS est appliquée, l'erreur relative comprend aussi l'erreur ajoutée par le modèle d'estimation de vitesse lorsque le procédé d'entraînement est interrompu. La figure 8 montre les résultats pour différentes personnes lorsque la stratégie intelligente GNSS est appliquée à tous les participants dans la base de donnée M3.

Si tous les participants sont pris en considération, l'erreur relative est autour de 6.5% lorsque le module récepteur GNSS est activé en continu selon la courbe $G1_{ON}$. Cela augmente à 7.5% une fois que la stratégie intelligente GNSS est appliquée selon la courbe G1 pour le module récepteur GNSS de la présente invention. D'autre part, si seuls les participants dont les enregistrements contenus pendant au moins 10 heures de données sont compris, l'erreur relative dans l'estimation de vitesse est sous 5% selon la courbe $G10_{ON}$ lorsque le module récepteur GNSS est activé 100% du temps. Après l'application de la stratégie intelligente GNSS, l'erreur relative est proche de 6% selon la courbe G10 avec une activation du module récepteur GNSS en-dessous de 10%. Avec ceci et comme représenté dans les figures précédentes, il est clair que la stratégie proposée de la présente invention peut atteindre une forte réduction dans l'utilisation ou activation du module récepteur GNSS en maintenant une erreur raisonnablement faible dans l'estimation de vitesse. De plus, la stabilité et le niveau de convergence du modèle RL paraît acceptable en comparaison au cas où le module récepteur GNSS est toujours activé (ON). La durée de l'activation successive du module récepteur GNSS peut donc être inférieure à 10% du temps total d'utilisation du dispositif de contrôle par l'algorithme de calcul de l'unité de calcul, c'est-à-dire avec une durée maximale de mise en fonction du module récepteur GNSS d'environ 5 heures, ledit module récepteur GNSS n'étant plus mis en fonction au-delà d'un temps total d'utilisation du dispositif de contrôle de 50 heures. Ainsi le dispositif de contrôle de l'instrument portable peut être alimenté par une petite batterie, telle qu'une batterie de montre-bracelet.

A partir de la description qui vient d'être faite, plusieurs variantes de réalisation de l'instrument portable muni du dispositif de contrôle d'une activité sportive ou de bien-être d'une personne et du procédé de mise en action du dispositif de contrôle sont possibles sans sortir du cadre de l'invention définie par les revendications suivantes. Une ou plusieurs mémoires non volatiles peuvent être prévues et susceptibles d'être détachées du dispositif de contrôle pour équiper un autre instrument portable avec toutes les données de mouvement ou profils de marche personnalisés enregistrés et dédiés à une personne. L'alimentation électrique de l'instrument portable peut être fournie par une batterie ou une cellule solaire ou un générateur thermoélectrique.

**Revendications**

1. Instrument portable (3) muni d'un dispositif de contrôle (10) ou de gestion d'une activité sportive ou de bien-être d'une personne (1) portant l'instrument portable (3), le dispositif de contrôle (10) comprenant un microcontrôleur (15), un capteur de mouvement (13), qui comprend au moins un accéléromètre tridimensionnel pour fournir un signal d'accéléromètre A(t) au microcontrôleur (15), un capteur de pression barométrique (14) pour fournir un signal de pression au microcontrôleur (15) et un module récepteur GNSS (11) relié au microcontrôleur (15),

    le microcontrôleur (15) étant agencée pour activer le module récepteur GNSS (11) pendant un temps d'activation défini suite à des variations de mouvement détectées par le capteur de mouvement (13) s'écartant de données de mouvement ou profils de marche connus afin de déterminer au moins une vitesse de référence de la personne, et pour désactiver le module récepteur GNSS (11) après le temps d'activation pendant un temps de désactivation plus grand que le temps d'activation, le temps d'activation du module récepteur GNSS étant inférieur de près de 5 fois au temps de désactivation du module récepteur GNSS, l'instrument portable (3) étant agencé pour effectuer une estimation de vitesse ou de cadence du déplacement d'une personne (1) portant l'instrument portable (3) par un algorithme de calcul dans le microcontrôleur (15), afin de contrôler également l'activation ou la désactivation du module récepteur GNSS (11),
    **caractérisé en ce que** le microcontrôleur (15) est agencé pour segmenter le signal d'accéléromètre et le signal de pression toutes les secondes à l'aide d'une fenêtre mobile d'une première durée avec un chevauchement d'une seconde durée avec une fenêtre mobile successive pour fournir une accélération segmentée et un signal de pression segmenté de manière à déterminer dans le microcontrôleur (15) si le modèle ou profil de marche détecté est un nouveau modèle ou profil non encore mémorisé.

2. Instrument portable (3) selon la revendication 1, dans lequel le dispositif de contrôle (10) comprend au moins une mémoire (16) reliée au microcontrôleur (15) pour mémoriser au moins des données de vitesse et différents profils personnalisés de marche ou course de la personne portant l'instrument portable (3).

3.  Instrument portable (3) selon la revendication 2, dans lequel la mémoire (16) reliée au microcontrôleur (15) comprend un algorithme de calcul enregistré pour l'estimation de vitesse ou de cadence du déplacement d'une personne (1) portant l'instrument portable (3).

4.  Instrument portable (3) selon la revendication 1, **caractérisé en ce que** le microcontrôleur (15) comprend un premier compteur en liaison à un premier seuil de commutation pour déterminer le temps d'activation du module récepteur GNSS (11) et un second compteur en liaison à un second seuil de commutation pour déterminer le temps de désactivation du module récepteur GNSS (11).

5.  Instrument portable (3) selon la revendication 1, **caractérisé en ce que** le capteur de mouvement est un capteur inertiel de mouvement à 9 axes ayant un accéléromètre triaxial, un gyroscope triaxial et un capteur magnétique triaxial ou un capteur inertiel de mouvement à 10 axes ayant un accéléromètre triaxial, un gyroscope triaxial, un capteur magnétique triaxial et un baromètre.

6.  Instrument portable (3) selon la revendication 1, qui est sous la forme d'une montre-bracelet (3) alimentée par une batterie.

7.  Procédé de gestion d'une activité sportive ou de bien-être d'une personne (1) portant un instrument portable (3), qui est muni d'un dispositif de contrôle (10), le dispositif de contrôle (10) comprenant un un capteur de mouvement (13), qui comprend au moins un accéléromètre tridimensionnel pour fournir un signal d'accéléromètre A(t) au microcontrôleur (15), un capteur de pression barométrique (14) pour fournir un signal de pression au microcontrôleur (15) et un module récepteur GNSS (11) relié au microcontrôleur (15),

    le procédé comprenant les étapes de :

        - activer le module récepteur GNSS (11) pendant un temps d'activation défini suite à des variations de mouvement détectées par le capteur de mouvement (13) s'écartant de données de mouvement ou profils de marche connus,
        - déterminer au moins une distance ou vitesse de référence de la personne suite à l'activation du module récepteur GNSS (11) par réception de signaux GPS de satellites (22), et
        - désactiver le module récepteur GNSS (11) après le temps d'activation pendant un temps de désactivation plus grand que le temps d'activation, le temps d'activation du module récepteur GNSS étant inférieur de près de 5 fois au temps de désactivation du module récepteur GNSS,

    où une estimation de vitesse ou de cadence du déplacement d'une personne (1) portant l'instrument portable (3) est effectuée par un algorithme de calcul dans le microcontrôleur (15) afin de contrôler également l'activation ou la désactivation du module récepteur GNSS (11),
    **caractérisé en ce que** le signal d'accéléromètre et le signal de pression sont segmentés toutes les secondes à l'aide d'une fenêtre mobile d'une première durée avec un chevauchement d'une seconde durée avec une fenêtre mobile successive pour fournir une accélération segmentée et un signal de pression segmenté de manière à déterminer dans le microcontrôleur (15) si le modèle ou profil de marche détecté est un nouveau modèle ou profil non encore mémorisé.

8.  Procédé de gestion selon la revendication 7, **caractérisé en ce que** la première durée est égale à 6 secondes et la seconde durée est égale à 5 secondes selon un cadencement d'un oscillateur du microcontrôleur (15).

9.  Procédé de gestion selon la revendication 7, pour lequel le microcontrôleur (15) comprend un premier compteur (56) pour déterminer un temps d'activation du module récepteur GNSS (11) et un second compteur (58) pour déterminer un temps de désactivation du module récepteur GNSS (11), **caractérisé en ce qu'**à la suite de la détermination du modèle ou profil de marche, il est contrôlé si le module récepteur GNSS (11) est déjà dans un état activé ou dans un état désactivé, **en ce que** dans le cas où le module récepteur GNSS (11) est déjà dans un état activé, un contrôle de l'état du premier compteur est effectué pour déterminer si l'état du premier compteur est en dessous d'un premier seuil minimum si oui le premier compteur est incrémenté et les précédentes étapes du procédé sont répétées, dans le cas contraire au-dessus du premier seuil, il est contrôlé si une décision de commutation du module récepteur GNSS (11) doit intervenir afin de déterminer si le module récepteur GNSS (11) doit être désactivé, et **en ce que** dans le cas où le module récepteur GNSS (11) est déjà dans un état désactivé, un contrôle de l'état du second compteur est effectué pour déterminer si l'état du second compteur est en dessous d'un second seuil minimum si oui le second compteur est incrémenté et les précédentes étapes du procédé sont répétées, dans le cas contraire au-dessus du second seuil, il est contrôlé si une décision de commutation du module récepteur GNSS (11) doit intervenir afin de déterminer si le module récepteur GNSS (11) doit être activé.

10. Procédé de gestion selon la revendication 9, **caractérisé en ce que** le temps d'activation du module récepteur GNSS (11) défini par le premier compteur (56) est fixé à 2 minutes, et **en ce que** le temps de désactivation du module récepteur GNSS (11) défini par le second compteur (58) est plus grand que 10 minutes et dépend de nouveaux modèles ou profils de marche détectés

11. Procédé de gestion selon la revendication 7, **caractérisé en ce qu'**en mettant le dispositif de contrôle (10) en fonction lorsque l'instrument portable (3) est utilisé par la personne (1), la durée de l'activation successive du module récepteur GNSS (11) est inférieure à 10% du temps total d'utilisation du dispositif de contrôle (10) par l'algorithme de calcul du microcontrôleur (15), c'est-à-dire avec une durée maximale de mise en fonction du module récepteur GNSS (11) d'environ 5 heures, ledit module récepteur GNSS (11) n'étant plus mis en fonction si l'instrument portable (3) est alimenté par une batterie au-delà d'un temps total d'utilisation du dispositif de contrôle (10) de 50 heures.

**Patentansprüche**

1. Tragbares Instrument (3), versehen mit einer Vorrichtung (10) zum Steuern oder Verwalten einer Sport- oder Wellnessaktivität einer Person (1), die das tragbare Instrument (3) trägt, die Steuervorrichtung (10) umfassend einen Mikrocontroller (15), einen Bewegungssensor (13), der mindestens einen dreidimensionalen Beschleunigungsmesser zum Bereitstellen eines Beschleunigungsmessersignals A(t) an den Mikrocontroller (15) umfasst, einen barometrischen Drucksensor (14) zum Bereitstellen eines Drucksignals an den Mikrocontroller (15), und ein GNSS-Empfängermodul (11), das mit dem Mikrocontroller verbunden ist, wobei der Mikrocontroller (15) so angeordnet ist, dass er das GNSS-Empfängermodul (11) für eine Aktivierungszeit aktiviert, die gemäß Bewegungsänderungen definiert ist, die von dem Bewegungssensor (13) erfasst werden und von bekannten Bewegungsdaten oder Gehprofilen abweichen, um mindestens eine Referenzgeschwindigkeit für die Person zu bestimmen, und das GNSS-Empfängermoduls (11) nach der Aktivierungszeit für eine Deaktivierungszeit deaktiviert, die größer als die Aktivierungszeit ist, wobei die Aktivierungszeit des GNSS-Empfängermoduls ungefähr fünfmal kürzer als die Deaktivierungszeit des GNSS-Empfängermoduls ist, wobei das tragbare Instrument (3) dafür ausgelegt ist, die Geschwindigkeit oder das Tempo der Bewegung einer Person (1), die das tragbare Instrument (3) trägt, unter Verwendung eines Berechnungsalgorithmus in dem Mikrocontroller (15) zu schätzen, um auch die Aktivierung oder Deaktivierung des GNSS-Empfängermoduls (11) zu steuern, **dadurch gekennzeichnet, dass** der Mikrocontroller (15) so ausgelegt ist, dass er das Beschleunigungsmessersignal und das Drucksignal jede Sekunde unter Verwendung eines mobilen Fensters mit einer ersten Dauer mit einer Überlappung einer zweiten Dauer mit einem nachfolgenden mobilen Fenster segmentiert, um eine segmentierte Beschleunigung und ein segmentiertes Drucksignal bereitzustellen, um in dem Mikrocontroller (15) zu bestimmen, ob das erfasste Gehmodell oder -profil ein neues Modell oder Profil ist, das noch nicht gespeichert wurde.

2. Tragbares Instrument (3) nach Anspruch 1, wobei die Steuervorrichtung (10) mindestens einen Speicher (16) umfasst, der mit dem Mikrocontroller (15) verbunden ist, um zumindest Geschwindigkeitsdaten und verschiedene individuell angepasste Geh- oder Laufprofile der das tragbare Instrument (3) tragenden Person abzuspeichern.

3. Tragbares Instrument (3) nach Anspruch 2, wobei der mit dem Mikrocontroller (15) verbundene Speicher (16) einen aufgezeichneten Berechnungsalgorithmus zum Schätzen der Geschwindigkeit oder des Tempos der Bewegung einer Person (1), die das tragbare Instrument (3) trägt, umfasst.

4. Tragbares Instrument (3) nach Anspruch 1, **dadurch gekennzeichnet, dass** der Mikrocontroller (15) einen ersten Zähler, der sich mit einem ersten Schaltschwellenwert verbindet, um die Aktivierungszeit des GNSS-Empfängermoduls (11) zu bestimmen, und einen zweiten Zähler, der sich mit einem zweiten Schaltschwellenwert verbindet, um die Deaktivierungszeit des GNSS-Empfängermoduls (11) zu bestimmen, umfasst.

5. Tragbares Instrument (3) nach Anspruch 1, **dadurch gekennzeichnet, dass** der Bewegungssensor ein Trägheitsbewegungssensor mit 9 Achsen ist, aufweisend einen dreiachsigen Beschleunigungsmesser, einen dreiachsigen Kreisel und einen dreiachsigen Magnetsensor, oder ein Trägheitsbewegungssensor mit 10 Achsen ist, aufweisend einen dreiachsigen Beschleunigungsmesser, einen dreiachsigen Kreisel, einen dreiachsigen Magnetsensor und ein Barometer.

6. Tragbares Instrument (3) nach Anspruch 1, das die Form einer Armbanduhr (3) hat, die von einer Batterie gespeist wird.

7. Verfahren zum Verwalten einer Sport- oder Wellnessaktivität einer Person (1), die ein tragbares Instrument (3) trägt, das mit einer Steuervorrichtung (10) versehen ist, die Steuervorrichtung (10) umfassend einen Mikrocontroller, einen Bewegungssensor (13), der zumindest einen dreidimensionalen Beschleunigungsmesser zum Bereitstellen eines Beschleunigungsmessersignals A(t) an den Mikrocontroller (15) umfasst, einen barometrischen Drucksensor (14) zum Bereitstellen eines Drucksignals an den Mikrocontroller (15 und ein GNSS-Empfängermodul (11), das mit dem Mikrocontroller (15) verbunden ist, das Verfahren umfassend die folgenden Schritte:

- Aktivieren des GNSS-Empfangsmoduls (11) für eine Aktivierungszeit, die gemäß Bewegungsänderungen definiert ist, die durch den Bewegungssensor (13) erfasst werden und von bekannten Bewegungsdaten oder Gehprofilen abweichen,
- Bestimmen zumindest einer Referenzdistanz oder - geschwindigkeit für die Person im Anschluss an die Aktivierung des GNSS-Empfängermoduls (11) durch Empfang von GPS-Signalen von Satelliten (22), und
- Deaktivieren des GNSS-Empfängermoduls (11) nach der Aktivierungszeit für eine Deaktivierungszeit, die größer ist als die Aktivierungszeit, wobei die Aktivierungszeit des GNSS-Empfängermoduls ungefähr fünfmal kürzer ist als die Deaktivierungszeit des GNSS-Empfängermoduls,

wobei eine Schätzung der Geschwindigkeit oder des Tempos der Bewegung einer Person (1), die das tragbare Instrument (3) trägt, unter Verwendung eines Berechnungsalgorithmus in dem Mikrocontroller (15) durchgeführt wird, um auch die Aktivierung oder Deaktivierung des GNSS-Empfängermoduls (11) zu steuern, **dadurch gekennzeichnet, dass** das Beschleunigungsmessersignal und das Drucksignal jede Sekunde unter Verwendung eines mobilen Fensters mit einer ersten Dauer mit einer Überlappung einer zweiten Dauer mit einem aufeinanderfolgenden mobilen Fenster segmentiert werden, um eine segmentierte Beschleunigung und ein segmentiertes Drucksignal bereitzustellen, um in dem Mikrocontroller (15) zu bestimmen, ob das erfasste Gehmodell oder -profil ein neues Modell oder Profil ist, das noch nicht gespeichert wurde.

8. Verwaltungsverfahren nach Anspruch 7, **dadurch gekennzeichnet, dass** die erste Dauer gleich 6 Sekunden und die zweite Dauer gleich 5 Sekunden gemäß einer Zeitmessung eines Oszillators des Mikrocontrollers (15) ist.

9. Verwaltungsverfahren nach Anspruch 7, wobei der Mikrocontroller (15) einen ersten Zähler (56) zum Bestimmen einer Aktivierungszeit für das GNSS-Empfängermodul (11) und einen zweiten Zähler (58) zum Bestimmen einer Deaktivierungszeit für das GNSS-Empfängermodul (11) umfasst, **dadurch gekennzeichnet, dass**, nachdem das Gehmodell oder -profil bestimmt wurde, das GNSS-Empfangsmodul (11) darauf überprüft wird, ob es sich bereits in einem aktivierten Zustand oder in einem deaktivierten Zustand befindet, und dadurch, dass, wenn sich das GNSS-Empfängermodul (11) bereits in einem aktivierten Zustand befindet, der Zustand des ersten Zählers überprüft wird, um zu bestimmen, ob der Zustand des ersten Zählers unterhalb eines ersten Mindestschwellenwerts liegt; wenn dies der Fall ist, der erste Zähler inkrementiert wird und die vorherigen Schritte des Verfahrens wiederholt werden; ansonsten, wenn er über dem ersten Schwellenwert liegt, er darauf überprüft wird, ob eine Entscheidung zum Umschalten des GNSS-Empfängermoduls (11) getroffen werden muss, um zu bestimmen, ob das GNSS-Empfängermodul (11) deaktiviert werden muss, und dadurch, dass, wenn sich das GNSS-Empfängermodul (11) bereits in einem deaktivierten Zustand befindet, der Zustand des zweiten Zählers überprüft wird, um zu bestimmen, ob der Zustand des zweiten Zählers unter einem zweiten Mindestschwellenwert liegt; wenn dies der Fall ist, der zweite Zähler inkrementiert wird und die vorherigen Schritte des Verfahrens wiederholt werden; ansonsten, wenn er über dem zweiten Schwellenwert liegt, er darauf überprüft wird, ob eine Entscheidung zum Umschalten des GNSS-Empfängermoduls (11) getroffen werden muss, um zu bestimmen, ob das GNSS-Empfängermodul (11) aktiviert werden muss.

10. Verwaltungsverfahren nach Anspruch 9, **dadurch gekennzeichnet, dass** die Aktivierungszeit des GNSS-Empfängermoduls (11), definiert durch den ersten Zähler (56), auf 2 Minuten eingestellt wird, und dadurch, dass die Deaktivierungszeit des GNSS-Empfängermoduls (11), definiert durch den zweiten Zähler (58), größer als 10 Minuten ist und von neu erfassten Gehmodellen oder -profilen abhängt.

11. Verwaltungsverfahren nach Anspruch 7, **dadurch gekennzeichnet, dass** durch Einschalten der Steuervorrichtung (10), wenn das tragbare Instrument (3) von der Person (1) verwendet wird, die Dauer der nachfolgenden Aktivierung des GNSS-Empfängermoduls (11) weniger als 10 % der Gesamtzeit beträgt, in der die Steuervorrichtung (10) durch den Berechnungsalgorithmus des Mikrocontrollers (15) verwendet wird, was bedeutet, dass bei einer maximalen Betriebszeit des GNSS-Empfängermoduls (11) von ungefähr 5 Stunden das GNSS-Empfängermodul (11) nicht mehr eingeschaltet wird, wenn das tragbare Instrument (3) durch eine Batterie über eine Gesamtnutzungszeit der Steuervorrichtung (10) von mehr als 50 Stunden hinaus mit Strom versorgt wird.

**Claims**

1. A portable instrument (3) provided with a device (10) for controlling or managing a sports or wellness activity of a person (1) wearing the portable instrument (3), the control device (10) comprising a microcontroller (15), a motion sensor (13) which comprises at least one three-dimensional accelerometer for providing an accelerometer signal A(t) to the microcontroller (15), a barometric pressure sensor (14) for providing a pressure signal to the microcontroller (15), and a GNSS receiver module (11) connected to the microcontroller, the microcontroller (15) being arranged to activate the GNSS receiver module (11) for an activation time defined according to movement variations detected by the motion sensor (13) and deviating from known movement data or walking profiles in order to determine at least one reference velocity for the person, and to deactivate the GNSS receiver module (11) after the activation time for a deactivation time greater than the activation time, the activation time of the GNSS receiver module being approximately five times shorter than the deactivation time of the GNSS receiver module, the portable instrument (3) being designed to estimate the velocity or pace of the movement of a person (1) wearing the portable instrument (3) using a calculation algorithm in the microcontroller (15), in order to also control the activation or deactivation of the GNSS receiver module (11), **characterised in that** the microcontroller (15) is designed to segment the accelerometer signal and the pressure signal every second using a mobile window with a first duration with an overlap of a second duration with a successive mobile window to provide a segmented acceleration and a segmented pressure signal so as to determine in the microcontroller (15) if the walking model or profile detected is a new model or profile that has not yet been saved.

2. The portable instrument (3) according to claim 1, in which the control device (10) comprises at least one memory (16) connected to the microcontroller (15) for memorising at least velocity data and various customised walking or running profiles of the person wearing the portable instrument (3).

3. The portable instrument (3) according to claim 2, in which the memory (16) connected to the microcontroller (15) comprises a recorded calculation algorithm for estimating the velocity or the pace of the movement of a person (1) wearing the portable instrument (3).

4. The portable instrument (3) according to claim 1, **characterised in that** the microcontroller (15) comprises a first counter connecting to a first switching threshold to determine the activation time of the GNSS receiver module (11) and a second counter connecting to a second switching threshold to determine the deactivation time of the GNSS receiver module (11).

5. The portable instrument (3) according to claim 1, **characterised in that** the motion sensor is an inertial motion sensor with 9 axes having a triaxial accelerometer, a triaxial gyroscope and a triaxial magnetic sensor or an inertial motion sensor with 10 axes having a triaxial accelerometer, a triaxial gyroscope, a triaxial magnetic sensor, and a barometer.

6. The portable instrument (3) according to claim 1, which is in the form of a wristwatch (3) powered by a battery.

7. A method for managing a sports or wellness activity of a person (1) wearing a portable instrument (3), which is provided with a control device (10), the control device (10) comprising a microcontroller, a motion sensor (13) which comprises at least one three-dimensional accelerometer for providing an accelerometer signal A(t) to the microcontroller (15), a barometric pressure sensor (14) for providing a pressure signal to the microcontroller (15 and a GNSS receiver module (11) connected to the microcontroller (15), the method comprising the following steps:

   - activating the GNSS receiver module (11) for an activation time defined according to movement variations detected by the motion sensor (13) and deviating from known movement data or walking profiles,
   - determining at least one reference distance or velocity for the person subsequent to the activation of the GNSS receiver module (11) by reception of GPS signals from satellites (22), and
   - deactivating the GNSS receiver module (11) after the activation time for a deactivation time greater than the activation time, the activation time of the GNSS receiver module being approximately five times shorter than the deactivation time of the GNSS receiver module,

in which an estimation of the velocity or of the pace of the movement of a person (1) wearing the portable instrument (3) is made using a calculation algorithm in the microcontroller (15), in order to also control the activation or deactivation of the GNSS receiver module (11), **characterised in that** the accelerometer signal and the pressure signal are segmented every second using a mobile window with a first duration with an overlap of a second duration with a successive mobile window to provide a segmented acceleration and a segmented pressure signal so as to determine in the microcontroller (15) if the walking model or profile detected is a new model or profile that has not yet been saved.

8. The management method according to claim 7, **characterised in that** the first duration is equal to 6 seconds and the second duration is equal to 5 seconds according to a timing of an oscillator of the microcontroller (15).

9. The management method according to claim 7, in which the microcontroller (15) comprises a first counter (56) for determining an activation time for the GNSS receiver module (11) and a second counter (58) for determining a deactivation time for the GNSS receiver module (11), **characterised in that** after the walking model or profile has been determined, the GNSS receiver module (11) is checked as to whether it is already in an activated state or in a deactivated state, **in that** if the GNSS receiver module (11) is already in an activated state, the state of the first counter is checked to determine whether the state of the first counter is below a first minimum threshold; if this is the case, the first counter is incremented and the previous steps of the method are repeated; otherwise, if it is above the first threshold, it is checked as to whether a decision to switch the GNSS receiver module (11) must be made in order to determine whether the GNSS receiver module (11) must be deactivated, and **in that** if the GNSS receiver module (11) is already in a deactivated state, the state of the second counter is checked to determine whether the state of the second counter is below a second minimum threshold; if this is the case, the second counter is incremented and the previous steps of the method are repeated; otherwise, if it is above the second threshold, it is checked as to whether a decision to switch the GNSS receiver module (11) must be made in order to determine whether the GNSS receiver module (11) must be activated.

10. The management method according to claim 9, **characterised in that** the activation time of the GNSS receiver module (11) defined by the first counter (56) is set to 2 minutes, and **in that** the deactivation time of the GNSS receiver module (11) defined by the second counter (58) is greater than 10 minutes and depends on new walking models or profiles detected.

11. The management method according to claim 7, **characterised in that** by turning the control device (10) on when the portable instrument (3) is used by the person (1), the duration of the successive activation of the GNSS receiver module (11) is less than 10% of the total time in which the control device (10) is used by the calculation algorithm of the microcontroller (15), which means that with a maximum operating time of the GNSS receiver module (11) of approximately 5 hours, said GNSS receiver module (11) no longer being turned on if the portable instrument (3) is powered by a battery beyond a total usage time of the control device (10) of 50 hours.

EP 3 761 063 B1

# Fig. 1

# Fig. 2

14

Fig. 3

4

1

1

1

1

Fig. 4

22

10          11

Dispositif de contrôle

13 — Capteur de mouvement

14 — Baromètre

Unité de calcul

Module GNSS

Mémoire

Signaux GPS

12

15          16

## Fig. 5

Interruption mémoire tampon FIFO — 50

NON

OUI → Extraction de caractéristiques — 51 → Classification de profils de démarche — 52 → Activer GNSS? — 53

GNSS est ON → Mise à jour d'histogramme — 54 → TON > min TON? — 55

NON → TON++ — 56

OUI → Décision de commutation GNSS — 59 → Désactiver GNSS? — 62

OUI → Remise à zéro TOFF, TON — 63

NON → TON++

GNSS est OFF → TOFF > min TOFF? — 57

NON → TOFF++ — 58

OUI → Décision de commutation GNSS — 60 → Activer GNSS? — 61

OUI → Remise à zéro TOFF, TON — 63

NON → TOFF++

Fig. 6

EP 3 761 063 B1

## Fig. 7

Participant 1. GPS ON durant: 157.1833 min. 7.2114% du temps.

Fig. 8

**EP 3 761 063 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2018106319 A1
- WO 2012045484 A1
- US 7245254 B1
- US 20180356534 A1
- US 20130271314 A1